# EUROPEAN PATENT APPLICATION

(11) **EP 4 083 219 A1**
(43) Date of publication of application: **02.11.2022**
(21) Application number: 21170956.3
(22) Date of filing: 28.04.2021
(51) Int. Cl.: C12P 19/02, C12N 1/14, C12N 1/22, C12N 1/38

(54) **METHODS FOR CONVERTING A FEEDSTOCK COMPRISING TEXTILE AND/OR PAPER MATERIAL INTO VALUE-ADDED PRODUCT(S)**

(71) Applicant: Polli, Fabiola, 9712 LB Groningen (NL)
(72) Inventor: Polli, Fabiola, 9712 LB Groningen (NL)
(74) Representative: Arnold & Siedsma

(57) **Abstract**

The invention relates to a method for converting a feedstock comprising textile material and/or paper material into one or more value-added product(s), comprising the steps of:
(i) culturing fungal cells of the genus *Myceliophthora, Sporotrichum, Thielavia, Corynascus, Chrysosporium, Ctenomyces* or any mixture thereof, in a liquid growth medium comprising at least 0.3 wt% of lactose, at least 0.1 wt% of a nitrogen source, and less than 2 wt% of a non-inducing carbon source for a period to provide an induced fungal cell culture;
(ii) optionally subjecting a feedstock comprising textile material and/or paper material to a sterilization treatment to provide a sterilized feedstock;
(iii) contacting said feedstock or said sterilized feedstock with the induced fungal cell culture and a fermentation medium to form a fermentation mixture;
(iv) incubating the fermentation mixture under conditions allowing for conversion of the textile material into value-added product(s); and optionally
(v) isolating the value-added product(s) from said fermentation mixture.

## Description

The invention relates to methods for converting a feedstock comprising textile material and/or paper material into one or more value-added product(s).

Economic growth and a rising global population have resulted in an increasing fossil fuel demand and consequently an increasing emission of greenhouse gasses, having adverse effects on the environment.

In recent years, these increases have driven the development of renewable fuels. Renewable fuels, or biofuels, are fuels which are prepared from biomass such as crop residues or biomass-containing waste streams from industrial processes, such as waste from the food and drinks industry. Due to biofuels being derived from a renewable source, their net contribution in terms of greenhouse gas emission is much lower compared to fossil fuels.

Processed plant waste including molasses, sugar cane juice or fruit juice are regularly used as feedstock for the preparation of biofuels. These feedstocks have a high content of fermentable sugars, which can be converted directly by acidogenic bacteria into biofuels such as bioethanol or biodiesel (Prasad et al., Biochem. Cell. Arch. 2012. 12(2):155-160).

Textile and paper materials constitute a promising alternative feedstock to processed plant waste, because they are widely abundant as waste from the fashion and the pulp and paper industry and/or municipal waste.

These materials comprise cellulosic and/or lignocellulosic fibers that are typically extracted from plant material such as cotton, hemp and the like. Contrary to feedstocks comprising processed plant waste, cellulosic and lignocellulosic feedstocks are not readily convertible by most commonly used fermenting micro-organisms, because their structure is more complex.

In particular, lignocellulose is a complex composite material consisting primarily of the structural carbohydrates cellulose and hemicellulose (around 60-85 wt.% and 5 to 20 wt.% respectively) bonded to the organic polymer, lignin (around 0 to 15 wt.%) (L.C. Hao, R.M. Sheltami, in Natural Fibre Reinforced Vinyl Ester and Vinyl Polymer Composites, 2018).

Herein, the homopolymer cellulose is present in the form of crystalline microfibrils with low accessible surface area, which renders the polymer insoluble and therefore difficult to degrade by external sources. In addition, the cellulosic microfibrils are protected by a combination of hemicellulose and lignin, the latter being an organic polymer composed of the aromatic monomer units p-coumaryl alcohol, coniferyl alcohol and sinapyl alcohol. This protective layer should be removed or modified before conversion of cellulose to obtain fermentable sugar and/or ethanol is feasible (Andlar, et al. 2018. Eng. Life Sci 0:1-11).

Hence, the physical and chemical stability of lignocellulosic material hampers the efficient conversion of cellulose and hemicellulose into fermentable sugars and/or biofuel.

Accordingly, there is a need for a method for efficiently converting (ligno)cellulose-based textile materials and/or paper material into one or more value-added product(s), such as biofuels.

It has been found that a feedstock comprising textile material and/or paper material can be converted into one or more value-added product(s) such as fermentable sugars and/or biofuel, by using fungal cells of the genus *Myceliophthora, Sporotrichum, Thielavia, Corynascus, Chrysosporium* and/or *Ctenomyces* that are cultured using a unique liquid growth medium.

Accordingly, the invention relates to a method for converting a feedstock comprising textile material and/or paper material into one or more value-added product(s), comprising the steps of:
(i) culturing fungal cells of the genus *Myceliophthora, Sporotrichum, Thielavia, Corynascus, Chrysosporium, Ctenomyces* or any mixture thereof, in a liquid growth medium comprising at least 0.3 wt% of lactose, at least 0.1 wt% of a nitrogen source, and less than 2 wt% of a non-inducing carbon source for a period to provide an induced fungal cell culture;
(ii) optionally subjecting a feedstock comprising textile material and/or paper material to a sterilization treatment to provide a sterilized feedstock;
(iii) contacting said feedstock or said sterilized feedstock with the induced fungal cell culture and a fermentation medium to form a fermentation mixture;
(iv) incubating the fermentation mixture under conditions allowing for conversion of the textile material into value-added product(s); and optionally
(v) isolating the value-added product(s) from said fermentation mixture.

### Legend to the Figures

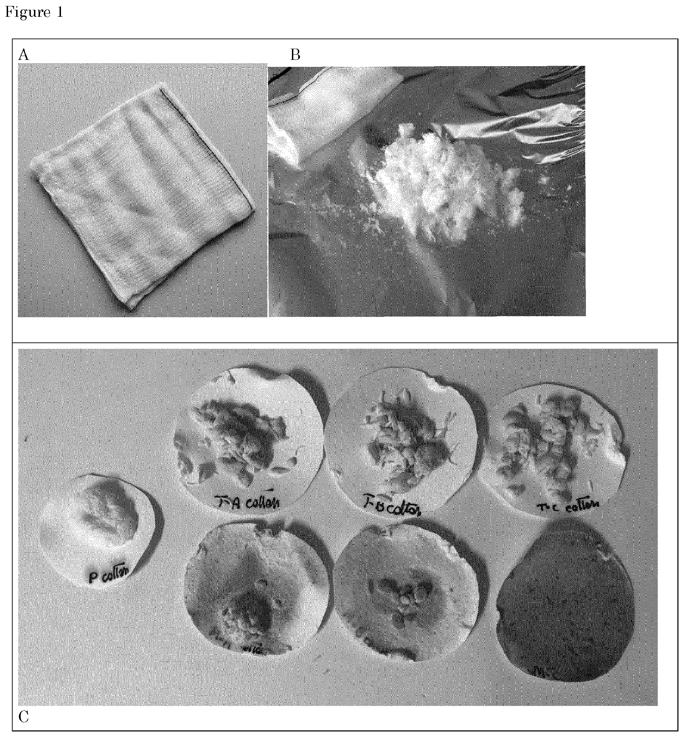

Figure 1; A: Feedstock comprising cotton; B: feedstock comprising cotton after grinding; C: feedstock comprising cotton after culturing with *Talaromyces emersonii* (upper three discs) and *Myceliophthora thermophila* (lower three discs) for 46 days. Left: control experiment after 46 days.

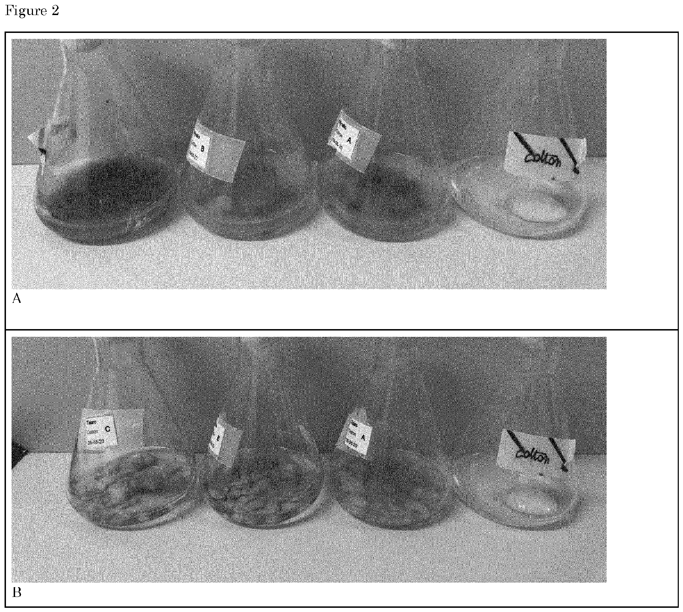

Figure 2; A: Fermentation mixture of feedstock comprising cotton and M. *thermophila* after 20 days (first three Erlenmeyer flasks). Right Erlenmeyer flask: control experiment after 20 days. B: Fermentation mixture of feedstock comprising cotton and *T. emersonii* after 20 days (first three Erlenmeyer flasks). Right Erlenmeyer flask: control experiment after 20 days.

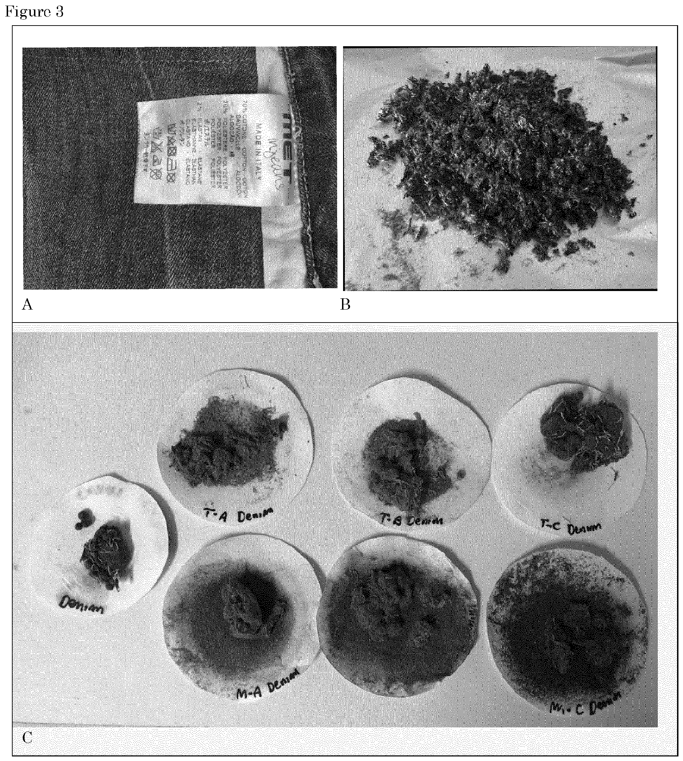

Figure 3; A: Feedstock comprising denim; B: feedstock comprising denim after grinding; C: feedstock comprising denim after culturing with *Talaromyces emersonii* (upper three discs) and *Myceliophthora thermophila* (lower three discs) for 46 days. Left: control experiment after 46 days.

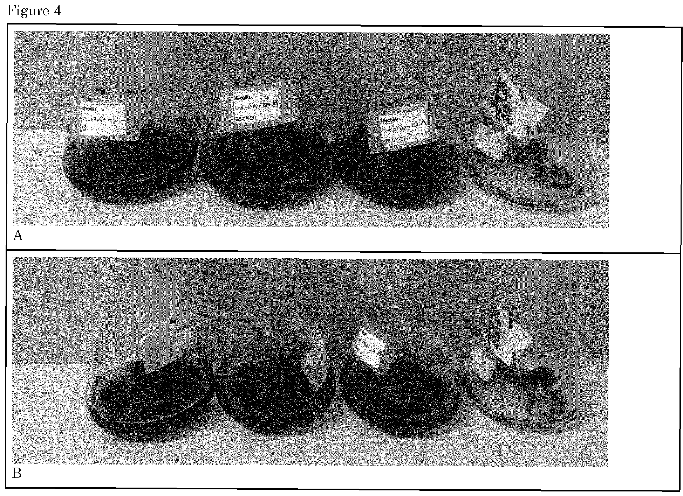

Figure 4; A: Fermentation mixture of feedstock comprising denim and *M*. *thermophila* after 20 days (first three Erlenmeyer flasks). Right Erlenmeyer flask: control experiment after 20 days. B: Fermentation mixture of feedstock comprising denim and *T. emersonii* after 20 days (first three Erlenmeyer flasks). Right Erlenmeyer flask: control experiment after 20 days.

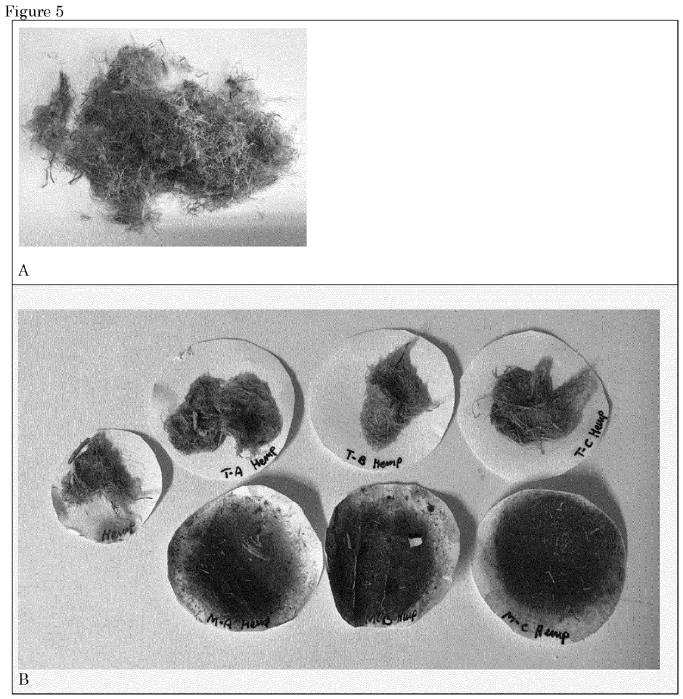

Figure 5; A: feedstock comprising bast fibers of hemp; B: feedstock comprising hemp after culturing with *Talaromyces emersonii* (upper three discs) and *Myceliophthora thermophila* (lower three discs) for 46 days. Left: control experiment after 46 days.

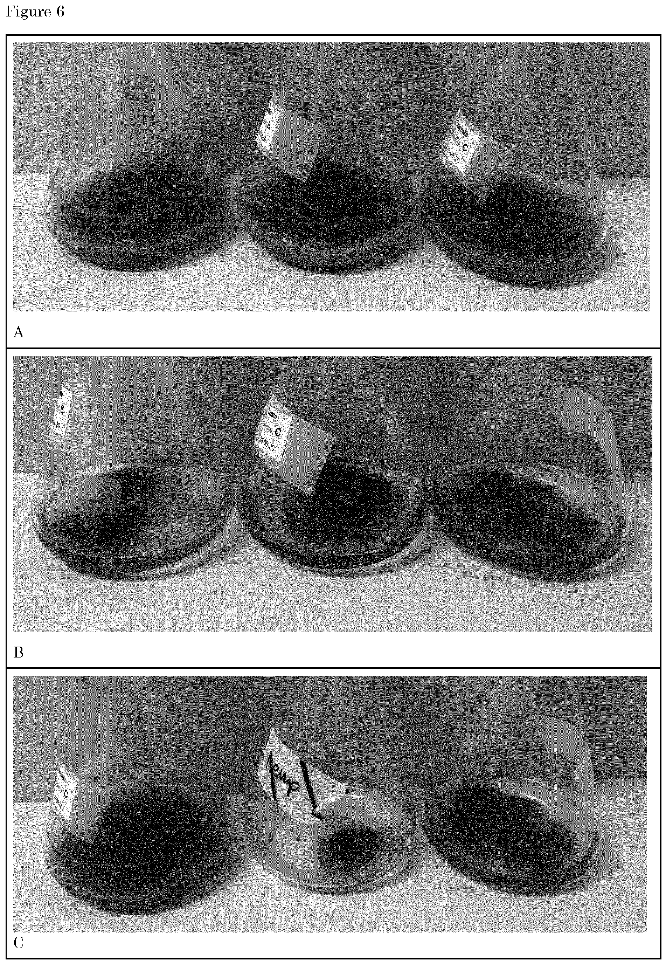

Figure 6; A: Fermentation mixture of feedstock comprising hemp and *M*. *thermophila* after 20 days (first three Erlenmeyer flasks). B: Fermentation mixture of feedstock comprising hemp and *T. emersonii* after 20 days (first three Erlenmeyer flasks). C: Left: Fermentation mixture of feedstock comprising hemp and *M*. *thermophila* after 20 days. Middle: Control experiment after 20 days; Right: Fermentation mixture of feedstock comprising hemp and *T. emersonii* after 20 day.

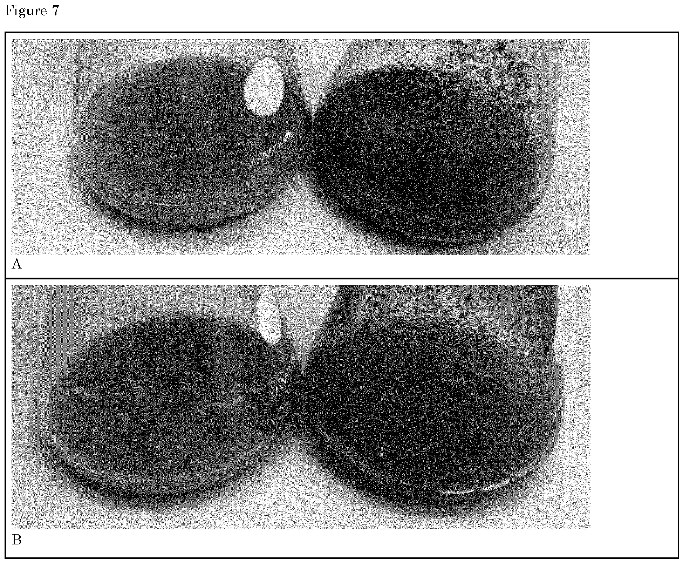

Figure 7; A: Fermentation mixture comprising bast fibers of hemp and *M*. *thermophila* at 0 days. B: Fermentation mixture comprising shaft fibers of hemp and *M. thermophila* at 0 days.

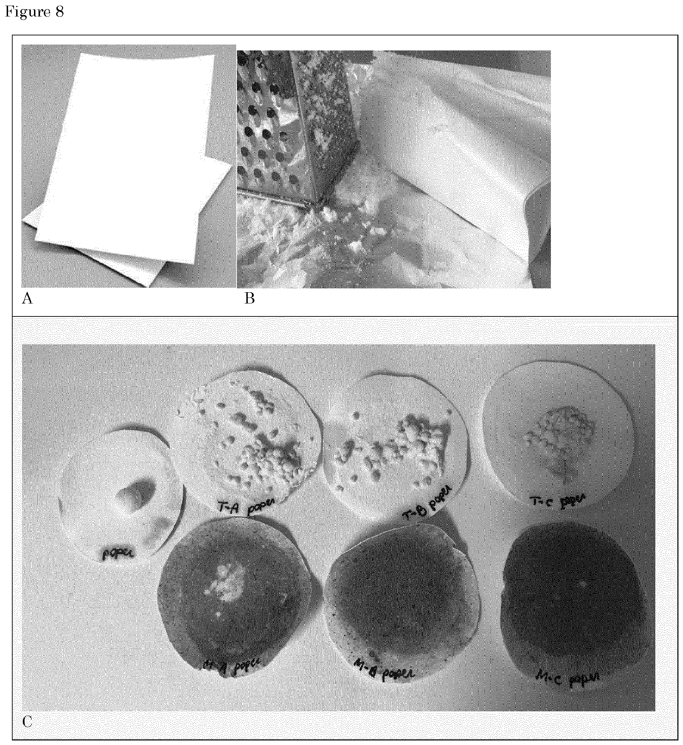

Figure 8; A: Feedstock comprising paper; B: Feedstock comprising paper after grinding; C: Feedstock comprising paper after culturing with *Talaromyces emersonii* (upper three discs) and *Myceliophthora thermophila* (lower three discs) for 46 days. Left: control experiment after 46 days.

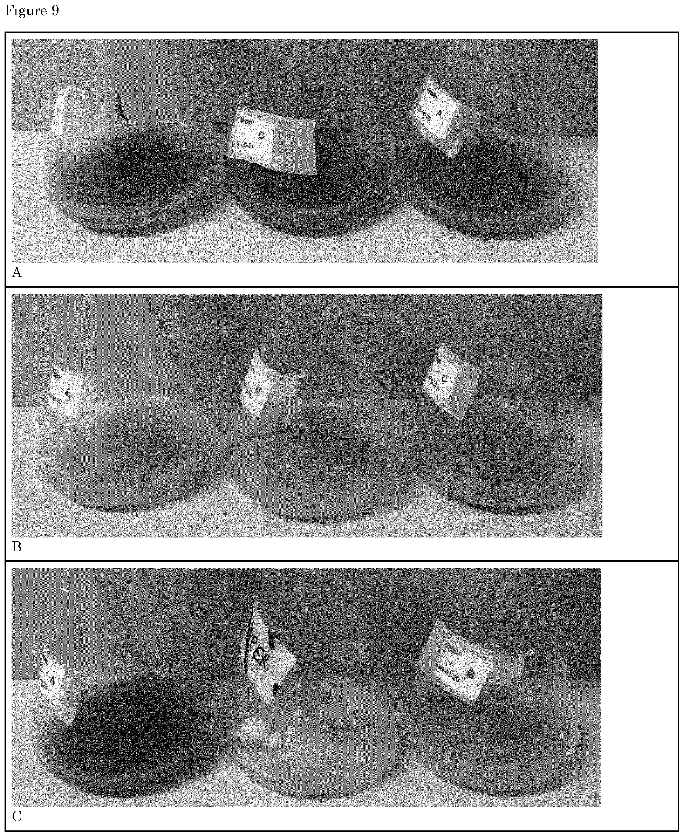

Figure 9; A: Fermentation mixture of feedstock comprising paper and *M*. *thermophila* after 20 days (first three Erlenmeyer flasks). B: Fermentation mixture of feedstock comprising paper and *T. emersonii* after 20 days (first three Erlenmeyer flasks). C: Left: Fermentation mixture of feedstock comprising paper and *M*. *thermophila* after 20 days. Middle: Control experiment after 20 days; Right: Fermentation mixture of feedstock comprising paper and *T. emersonii* after 20 day.

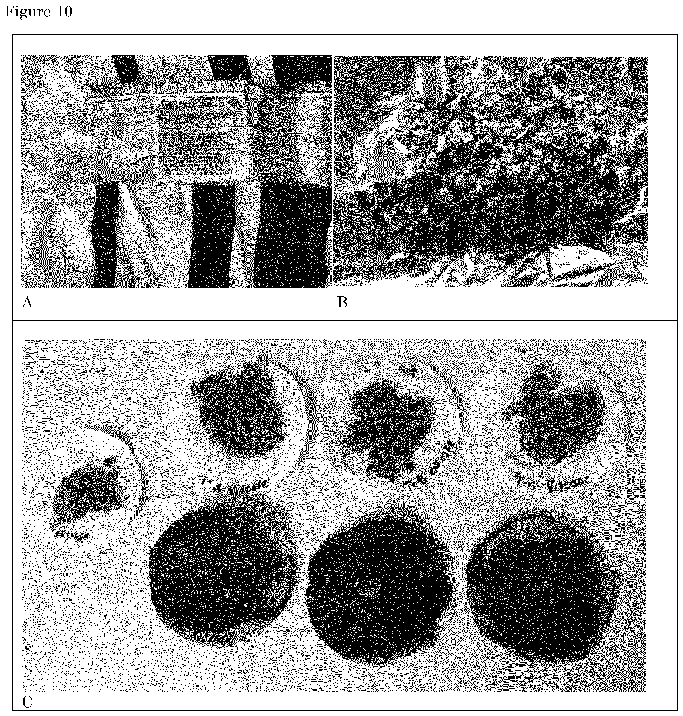

Figure 10; A: Feedstock comprising viscose; B: Feedstock comprising viscose after grinding; C: Feedstock comprising viscose after culturing with *Talaromyces emersonii* (upper three discs) and *Myceliophthora thermophila* (lower three discs) for 46 days. Left: control experiment after 46 days.

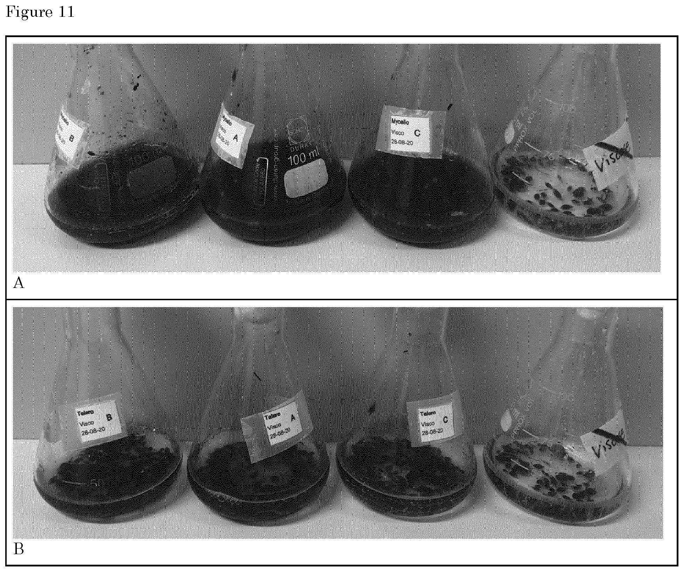

Figure 11; A: Fermentation mixture of feedstock comprising viscose and *M*. *thermophila* after 20 days (first three Erlenmeyer flasks). Right Erlenmeyer flask: control experiment after 20 days. B: Fermentation mixture of feedstock comprising viscose and *T. emersonii* after 20 days (first three Erlenmeyer flasks). Right Erlenmeyer flask: control experiment after 20 days.

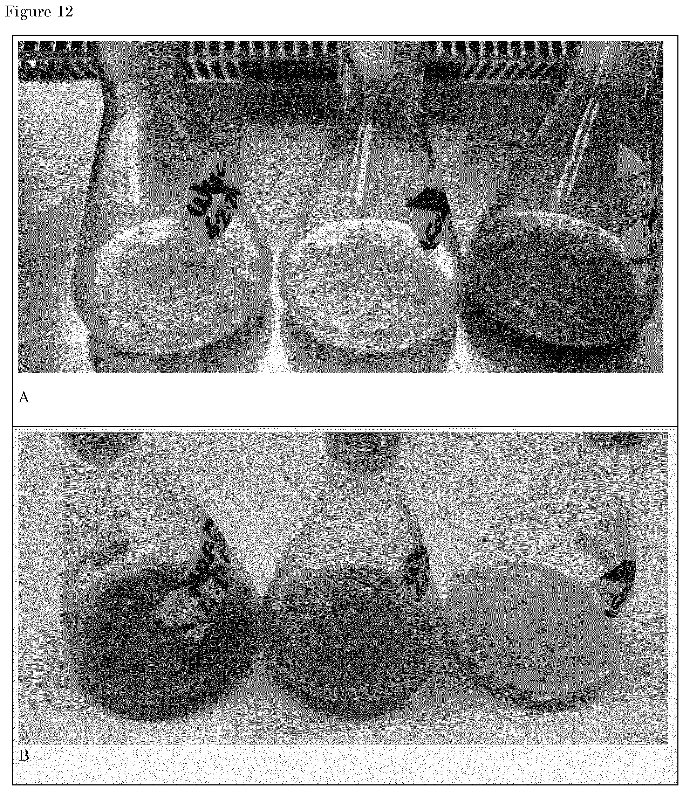

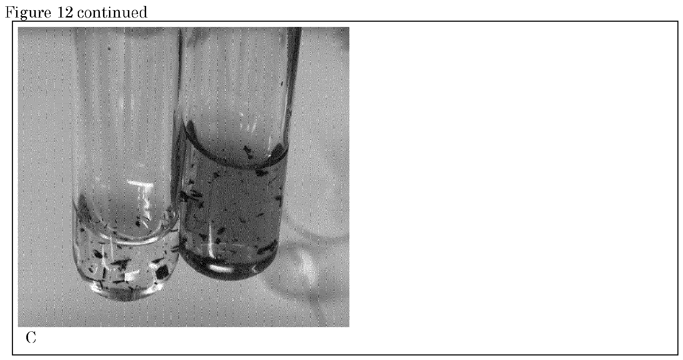

Figure 12; A: Fermentation mixture of feedstock comprising cotton and Penicillium Wisconsin (left); control (middle) and Penicillium NRRL right at day 0. B: Fermentation mixture of feedstock comprising cotton and Penicillium NRRL (left) Penicillium Wisconsin (center); control (right) at day 18. C: *Chaetomium thermophilum* in liquid growth medium.

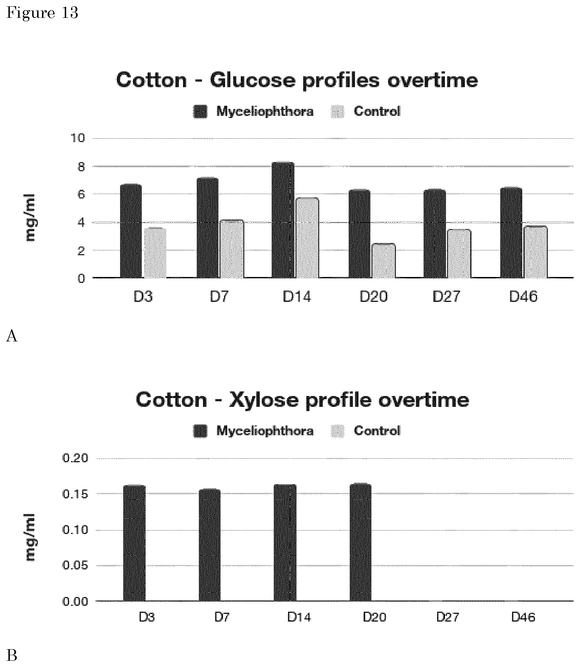

Figure 13; A: glucose levels in supernatant after 3, 7, 14, 20, 27 and 46 days from fermentation mixture comprising induced *M. thermophila* cells and a feedstock comprising cotton (black bars) and control (no induced fungal cells) (grey bars). B: xylose levels in culture supernatant after 3, 7, 14, 20, 27 and 46 days from fermentation mixture comprising induced *M. thermophila* cells and feedstock comprising cotton (black bars) and control (no induced fungal cells) (grey bars).

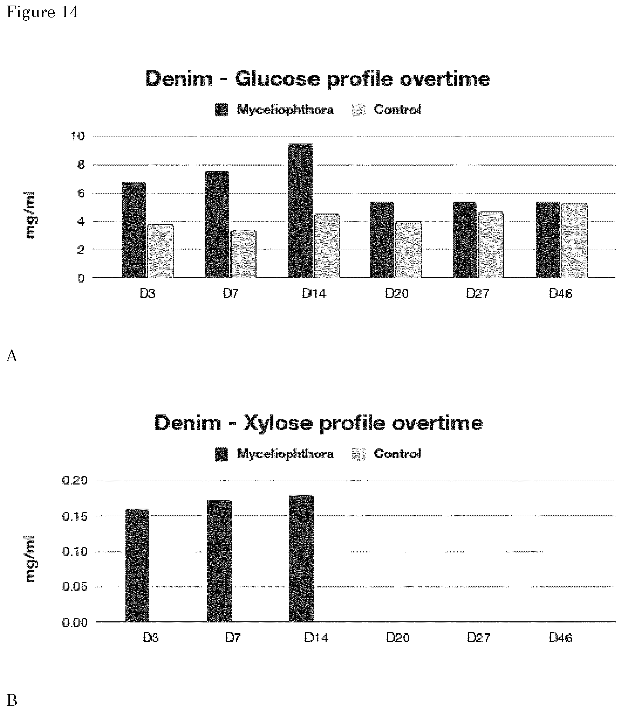

Figure 14; A: glucose levels in supernatant after 3, 7, 14, 20, 27 and 46 days from fermentation mixture comprising induced *M. thermophila* cells and feedstock comprising denim (black bars) and control (no induced fungal cells) (grey bars). B: xylose levels in culture supernatant after 3, 7, 14, 20, 27 and 46 days from fermentation mixture comprising induced *M. thermophila* cells and feedstock comprising denim (black bars) and control (no induced fungal cells) (grey bars).

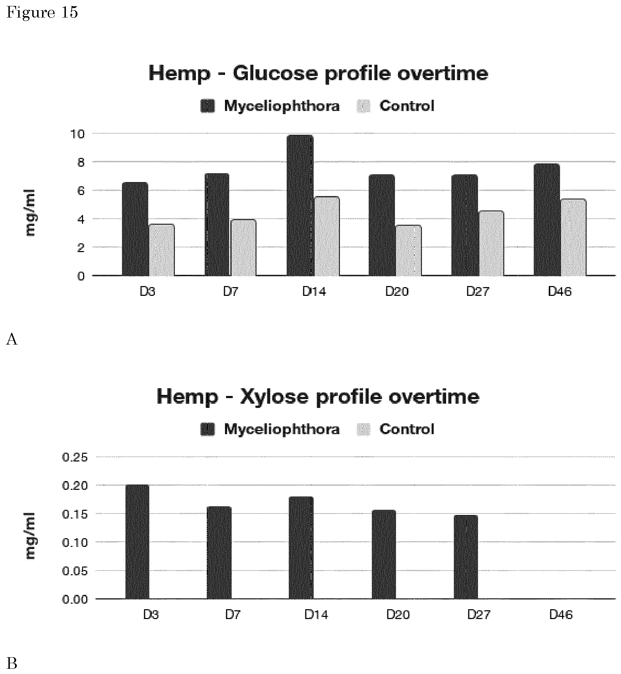

Figure 15; A: glucose levels in supernatant after 3, 7, 14, 20, 27 and 46 days from fermentation mixture comprising induced *M. thermophila* cells and feedstock comprising hemp (black bars) and control (no induced fungal cells) (grey bars). B: xylose levels in culture supernatant after 3, 7, 14, 20, 27 and 46 days from fermentation mixture comprising induced *M. thermophila* cells and feedstock comprising hemp (black bars) and control (no induced fungal cells) (grey bars).

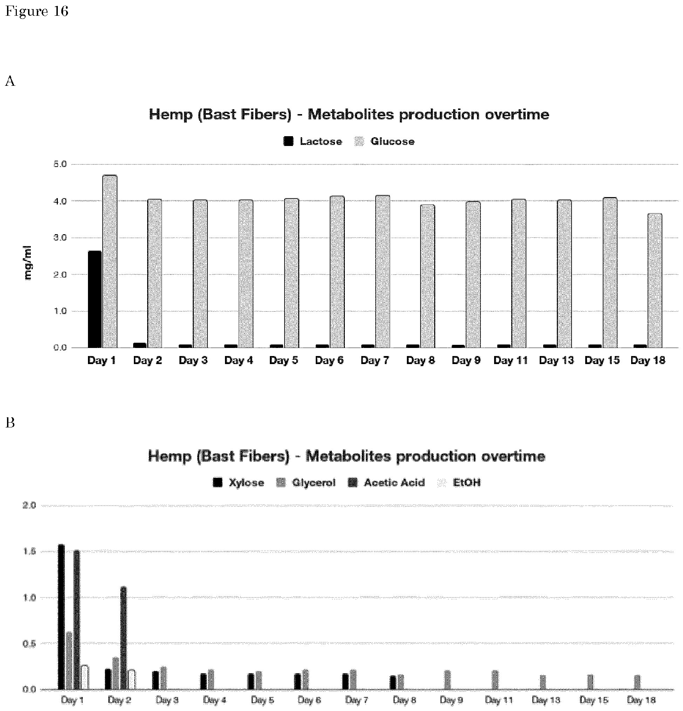

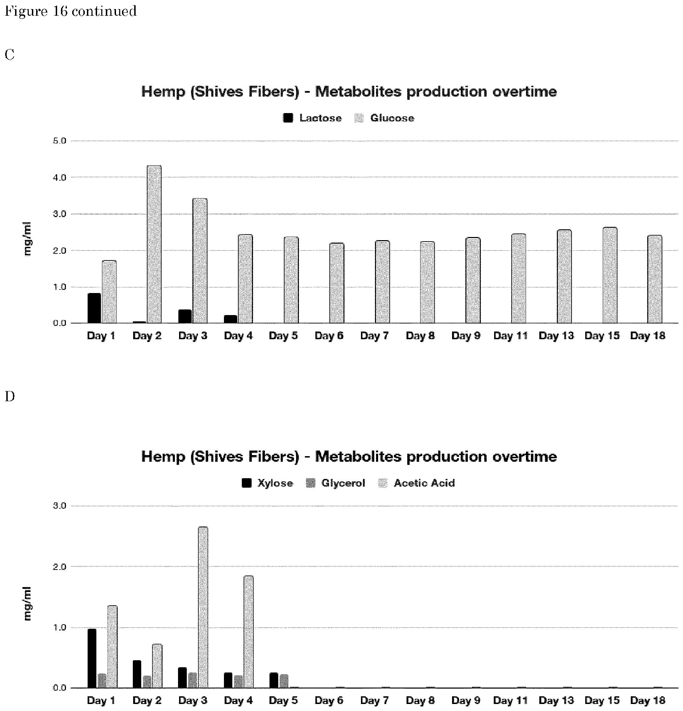

Figure 16; A: glucose (grey bars) and lactose (black bars) levels (g/mL) in supernatant after 1 to 18 days from fermentation mixture comprising induced *M*. *thermophila* cells and feedstock comprising hemp bast fibers. B: ethanol (EtOH), acetic acid, glycerol and xylose levels (mg/ml) in the supernatant of fermentation mixture comprising feedstock comprising bast fibers from hemp and induced *M*. *thermophila* cells. C: glucose (grey bars) and lactose (black bars) levels (g/mL) in supernatant after 1 to 18 days from fermentation mixture comprising induced *M*. *thermophila* cells and feedstock comprising hemp shive fibers. D: acetic acid, glycerol and xylose levels (mg/ml) in the supernatant of fermentation mixture comprising feedstock comprising shives fibers from hemp and induced *M*. *thermophila* cells.

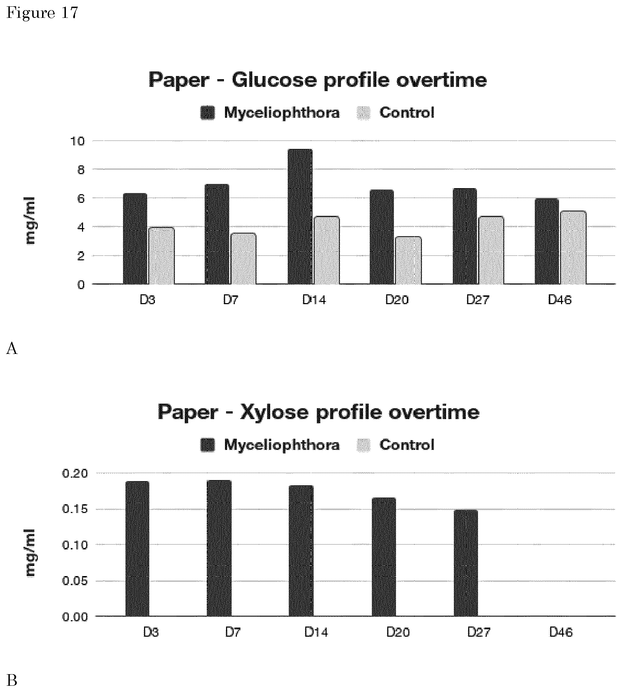

Figure 17; A: glucose levels in supernatant after 3, 7, 14, 20, 27 and 46 days from fermentation mixture comprising induced *M. thermophila* cells and feedstock comprising paper (black bars) and control (no induced fungal cells) (grey bars). B: xylose levels in culture supernatant after 3, 7, 14, 20, 27 and 46 days from fermentation mixture comprising induced *M. thermophila* cells and feedstock comprising paper (black bars) and control (no induced fungal cells) (grey bars).

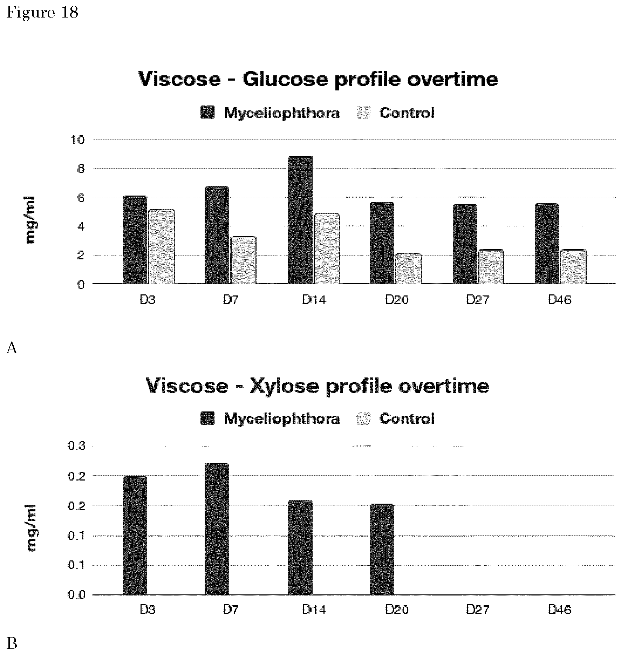

Figure 18; A: glucose levels in supernatant after 3, 7, 14, 20, 27 and 46 days from fermentation mixture comprising induced *M. thermophila* cells and feedstock comprising viscose (black bars) and control (no induced fungal cells) (grey bars). B: xylose levels in culture supernatant after 3, 7, 14, 20, 27 and 46 days from fermentation mixture comprising induced *M. thermophila* cells and feedstock comprising viscose (black bars) and control (no induced fungal cells) (grey bars).

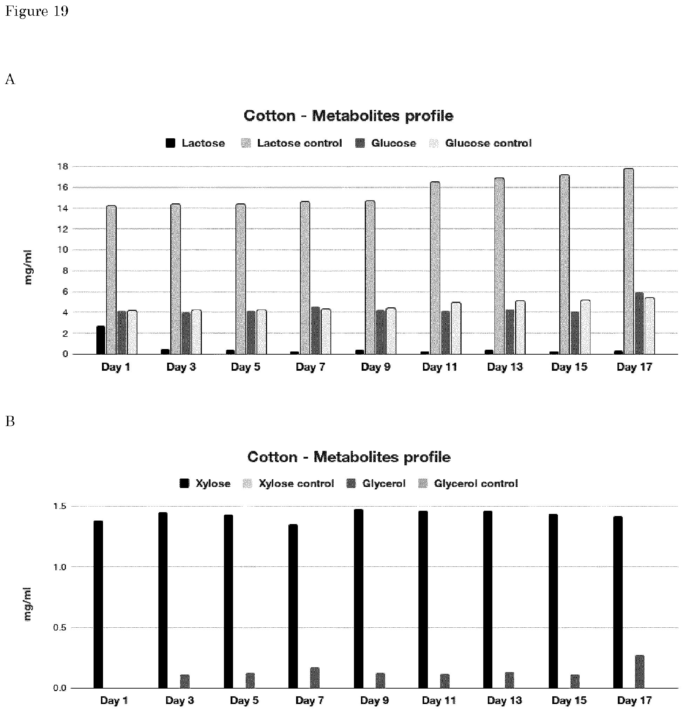

Figure 19; A: glucose and lactose levels in supernatant after 1, 3, 5 7, 9, 11, 13, 15 and 17 days from fermentation mixture comprising induced *M. thermophila* cells and feedstock comprising cotton and control (no induced fungal cells). B: xylose and glycerol levels in culture supernatant after 1, 3, 5 7, 9, 11, 13, 15 and 17 days from fermentation mixture comprising induced *M. thermophila* cells and feedstock comprising cotton and control (no induced fungal cells).

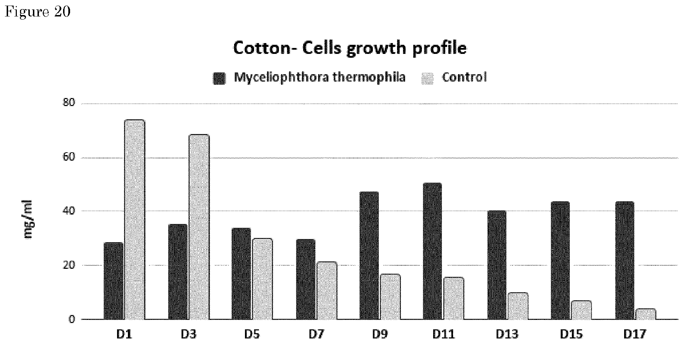

Figure 20; Protein levels in supernatant after 1, 3, 5, 7, 9, 11, 13, 15 and 17 days from fermentation mixture comprising induced *M. thermophila* cells and feedstock comprising cotton (black bars) and control (no feedstock comprising cotton) (grey bars).

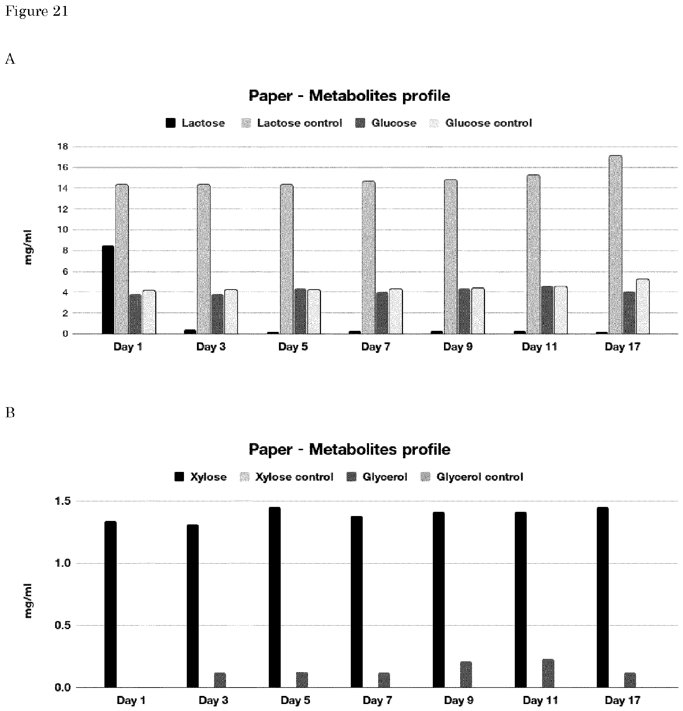

Figure 21; A: Glucose and lactose levels in supernatant after 1, 3, 5 7, 9, 11, 13, 15 and 17 days from fermentation mixture comprising induced *M. thermophila* cells and feedstock comprising paper and control (no induced fungal cells). B: xylose and glycerol levels in culture supernatant after 1, 3, 5 7, 9, 11, 13, 15 and 17 days from fermentation mixture comprising induced *M. thermophila* cells and feedstock comprising paper and control (no induced fungal cells).

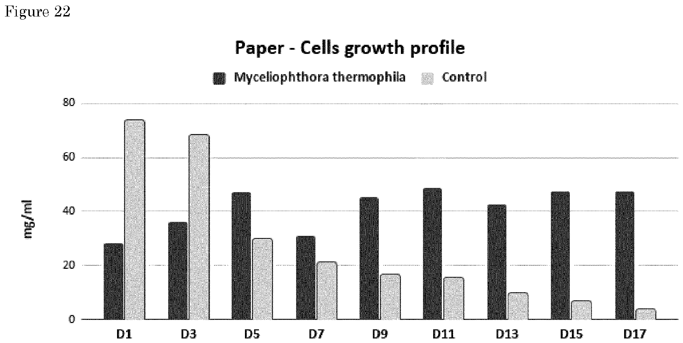

Figure 22; Protein levels in supernatant after 1, 3, 5, 7, 9, 11, 13, 15 and 17 days from fermentation mixture comprising induced *M. thermophila* cells and feedstock paper cotton (black bars) and control (no feedstock comprising cotton).

### Definitions

The term "or" as used herein is defined as "and/or" unless specified otherwise.

The term "a" or "an" as used herein is defined as "at least one" unless specified otherwise.

When referring to a noun *(e.g.* a compound, an additive, *etc.)* in the singular, the plural is meant to be included.

The term 'essentially free' is generally used herein to indicate that a substance is not present (below the detection limit achievable with analytical technology as available on the effective filing date) or present in such a low amount that it does not significantly affect the property of the product that is essentially free of said substance.

The term "textile material" is generally understood as meaning textile materials comprising natural fibers and/or synthetic fibers, comprising or consisting of cellulose. As is known, tensile strength of textile material is typically substantially unaffected by water, meaning that upon contact with water it doesn't substantially change in tensile strength. In the context of the application, the term "textile material" includes textile raw materials, textile structures produced therefrom, and finished products or products produced therefrom.

"Textile raw materials" as used herein refers to textile materials that are not subjected to one or more processes aimed to improve the physical properties of said textile materials in terms of smoothness, strength, shrinkage and the like, other than those processes required to separate textile fibers from their source material. Textile raw materials include unprocessed raw materials such hemp fibers obtained from the hemp plant and raw materials which are processed by various methods to linear, sheet-like and spatial structures. In particular, linear textile structures (such as yarns or threads), sheet-like textile structures (such as, for example, woven fabrics, braids, nonwovens and felts) and spatial textile structures (for example textile tubes, stockings or semi-finished textile products) can be produced from the raw materials.

"Finished textile materials" in the context of the present invention, refer to textile materials that have been converted by chemical and mechanical processes to improve the properties of the textile materials. Finishing processes include preparatory treatments used before additional treatment, such as bleaching prior to dyeing; treatments, such as glazing, to enhance appearance; sizing, affecting touch; and treatments adding properties to enhance performance, such as preshrinking. Finished textile materials may also contain non-textile materials.

As is known, the term "paper material" is generally used to refer to a material comprising natural cellulose fibers derived from a plant-based source, such as wood, grass or textile rags. As is known, paper material is typically not resistant to water, meaning that upon contact with water paper material substantially loses tensile strength. In the context of the present application, the term "paper material" may refer to paper pulp, paper structures prepared therefrom and finished paper products.

"Paper pulp" as used herein refers to an aqueous suspension of cellulose fibers derived from a vegetable source. Paper pulp is obtained by thermally, chemically, biologically and/or mechanically separating cellulose fibers from a vegetable source, such as wood. It may be subjected to one or more treatment steps to improve characteristics, e.g. paper pulp may be bleached to obtain a lighter color, preferably substantially white, or additives may be added to the paper pulp, such as fillers.

Paper pulp may be mechanically pressed to remove water and dried to form a dried paper material. Said dried paper material may be further processed to form a paper structure, such as a sheet-like paper structure, paperboard or cardboard.

"Finished paper products" refers to those paper products that have been subjected to one or more steps to improve their physical properties, for example, finished paper products may comprise a coating, such as a matte, semi-matte or gloss coating.

The term "value-added products" as used herein refers to products that have a commercial, scientific, nutritional and/or economical purpose.

The term "sterilization" as used herein refers to a step of removing micro-organisms without substantially degrading or disintegrating the material that is subjected to said sterilization.

The term "fermentable sugars" as used herein, refers to sugar molecules that can be converted into ethanol, acetic acid or glycerol by fermentation micro-organisms, such as yeast. Examples of fermentable sugars include glucose, xylose, fructose, maltose, sucrose and maltotriose.

The term "non-inducing carbon source" as used herein, refers to a carbon source that is not substantially capable of inducing a fungal cell to excrete extracellular enzymes, in particular cellulases, as can be established by determining the enzymatic activity of a fungal cell culture. Cellulase activity, as used herein, may for example be quantified by determining the total micromoles of reducing sugars released per milligram protein per minute. In this context, a fungus grown on a "non-inducing carbon source" as used herein produces less than 0.5 micromoles of reducing sugars per milligram protein per minute.

The term "cellulase" or "cellulase enzymes" is known in the art to refer to enzymes that catalyse the hydrolysis of the 1,4-glucosidic bonds connecting glucose monomer units in the cellulose polymer. Examples of cellulases include *endo*-1,4-β-D-glucanases (E.C. 3.2.1.4), *exo*-1,4-β-D-glucanases (E.C.3.2.1.74), cellobiohydrolases (E.C. 3.2.1.91), and β-glucosidase (E.C. 3.2.1.21). Endoglucanases hydrolyze accessible glucosidic bonds in the middle of the cellulose chain, while cellobiohydrolases release cellobiose from these chain ends processively. β-glucosidases hydrolyze cellobiose to glucose and, in doing so, minimize product inhibition of the cellobiohydrolases.

As used herein, the term "reducing sugar(s)" refers to a sugar molecule comprising a free aldehyde and/or hemiacetal moiety that is readily oxidised to carboxylic acids. Common test reagents are Fehlings reagent and Benedicts reagent.

In the context of the present application, the term "genetic modification" may be understood as meaning a fungal cell comprising a modification in its genome compared to the wild-type fungal cell of the same fungus. Said modification may be "genetically engineered", e.g. a result of targeted manipulation of the genome of said fungal cell or by random mutagenesis and selection or it may be a natural genetic variation, i.e. a genetic modification that is the result of natural evolution of the fungus. Examples of targeted manipulation include insertion of a gene encoding a protein of interest into the genome of the fungus, deletion of a gene encoding a protein of interest into the genome of the fungus or insertion of a gene that encodes antisense RNA or small interfering RNA. Suitable techniques for manipulation of a genome are well-known in the art. Random mutagenesis and selection refers to the process of creating a library of mutated strains, which are then screened for a desired altered phenotype. Said library of mutated strains may be created by natural or artificial means, including subjecting the fungal cell to irradiation or chemical mutagenesis.

### Methods

Currently, methods for converting textile material and/or paper material into value-added products utilize cellulose-degrading enzyme compositions.

An enzyme composition facilitating the conversion of a cellulosic and/or lignocellulosic feedstocks into fermentable sugars is described in WO 2013/177714. Herein a cellulose-degrading enzyme composition comprising one or more cellobiohydrolase or endoglucanase enzyme and at least one isolated Glycoside Hydrolase (GH) family 16 catalytic domain (GH16) polypeptide is described. However, the use of enzyme compositions, such as those described in WO 2013/177714 are associated with several disadvantages. For example, enzymes generally only work efficiently under optimum pH and temperature conditions, setting constraints to the method of employing the enzyme compositions. Further, the procedure of isolation and purification of enzymes may be costly and time consuming. Further, enzymes may require the presence of co-factors for their catalytic activity, further increasing complexity, costs and labor.

In addition, enzyme compositions, such as described in WO 2013/177714 A1 have a relatively limited substrate scope, since such compositions lack adaptivity to a feedstock and are thus only proficient in converting specific feedstocks that are comprised of materials that are converted by the enzymes present in the enzyme composition. Therefore, the use of an enzyme composition typically requires chemical or physical treatment of the feedstock prior to contacting it with the enzyme composition, in order to improve accessibility of fermentable substrates, such as cellulose, to the enzymes. Hence, in absence of such treatment, many textile and/or paper materials are not efficiently converted, because the enzymes present in the enzyme composition do not have sufficient access to the fermentable substrate.

Effective treatment steps, facilitating accessibility of lignocellulose to the fermentative enzymes, are steam explosion, wherein lignocellulosic material is treated with saturated steam at pressures between 0.69 and 4.83 MPa and temperatures of 160 - 260 °C (Cortes Tolalpa, L. (2018). Lignocellulose-degrading microbial consortia: Importance of synergistic interactions. Rijksuniversiteit Groningen, Chapter 1) and chemical treatment using organic solvents or strong acidic or alkaline conditions. However, steam explosion is associated with high processing costs and requires the use of large amounts of water and high temperatures, which poses a burden on the environment. Similarly, a chemical treatment requires the use of harsh, environmentally unfriendly materials and is associated with the formation of undesired inhibitory by-products.

Altogether, it is apparent that the requirement of treating a feedstock comprising textile material prior to fermentation renders the conversion of textile material into value-added products unsatisfactory. Such treatment of the feedstock not only increases the number of steps and production costs, the use of high temperatures, harmful chemicals and large amounts of water also renders the overall process less environmentally unfriendly. Furthermore, the yield is typically not optimal, because of degradation of part of the feedstock during the treatment conditions. Hence, there is a need to develop an improved method for converting a feedstock comprising textile material into one or more value-added products, wherein one or more of the above drawbacks are overcome.

The inventor found that using a fungal cell of any of the genera *Myceliophthora, Sporotrichum, Thielavia, Corynascus, Chrysosporium* and/or *Ctenomyces* when cultured in a liquid growth medium comprising at least 0.3 wt% of lactose, at least 0.1 wt% of a nitrogen source, and less than 2 wt% of a non-inducing carbon source allows the conversion of feedstocks comprising textile and/or paper material into value-added products, such as fermentable sugars and/or bioethanol without requiring harsh and environmentally unfriendly chemicals and/or conditions.

The invention therefore relates to a method for converting a feedstock comprising textile material and/or paper material into one or more value-added product(s), comprising the steps of:
(i) culturing fungal cells of the genus *Myceliophthora, Sporotrichum, Thielavia, Corynascus, Chrysosporium, Ctenomyces* or any mixture thereof, in a liquid growth medium comprising at least 0.3 wt% of lactose, at least 0.1 wt% of a nitrogen source, and less than 2 wt% of a non-inducing carbon source to provide an induced fungal cell culture;
(ii) optionally subjecting a feedstock comprising textile material and/or paper material to a sterilization treatment to provide a sterilized feedstock;
(iii) contacting said feedstock or said sterilized feedstock with the induced fungal cell culture and a fermentation medium to form a fermentation mixture;
(iv) incubating the fermentation mixture under conditions allowing for conversion of the textile material into value-added product(s); and optionally
(v) isolating the value-added product(s) from said fermentation mixture.

Without wishing to be bound by any theory, it is believed that an induced fungal cell culture comprising induced fungal cells of the genera *Myceliophthora, Sporotrichum, Thielavia, Corynascus, Chrysosporium* and/or *Ctenomyces* are capable of producing a mixture of enzymes is particularly suitable for converting feedstocks comprising textile material and/or paper material into value-added products. In particular, it is believed that said induced fungal cell culture excretes cellulases that are involved in conversion of cellulose into fermentable sugars and/or ethanol, and typically one or more of ligninolytic enzymes that catalyse the oxidation of aromatic substrates thereby modifying the structure of lignin and/or hemicellulases involved in degradation of hemicellulosic material. Accordingly, with the method according to the invention, feedstocks comprising textile material and/or paper material may be converted into value-added products without subjecting said feedstock to harsh chemical or physical conditions that pose a significant burden on the environment and complicate the method by requiring additional steps and materials, such as chemicals or complex equipment.

Therefore, in one aspect, the invention relates to a method wherein no organic solvents, including ionic liquids and/or conditions comprising a pH of at least 9 or less than 3, are used.

Furthermore, the induced fungal cell culture is adaptive and therefore capable of converting a broad scope of feedstocks comprising textile material and/or paper material.

The inventor realized that the method according to the invention is particularly suitable in converting a feedstock comprising a textile material into one or more value-added product(s).

As the population grows also the apparel consumption is estimated to increase to 102 million tons by 2030. About 85% thereof ends up as textile waste, which is typically incinerated or dumped in landfills where it takes 200 years or more to degrade. As it decomposes, toxic chemicals and dyes may leach into the groundwater and soil. Further, decomposition of textile is associated with emission of methane, a greenhouse gas that is far more potent than carbon dioxide, into the atmosphere. Hence, by using textile material as a feedstock for use in the method according to the invention, advantageously emission of environmentally unfriendly components into the environment may be avoided.

The feedstock for use in the method according to the invention therefore advantageously comprises one or more textile materials. Said feedstock may comprise textile raw materials, textile structures produced therefrom, finished products or products produced therefrom and combinations thereof. Said textile material may thus comprise natural fibers, synthetic fibers or a combination thereof. Good results have been obtained with a method according to the invention wherein the feedstock comprises one or more textile materials selected from the group consisting of cotton, denim, hemp, viscose, or a combination thereof.

In a further preferred embodiment, the method according to the invention preferably comprises a feedstock comprising a textile material, wherein the textile material comprises natural fibers. Said natural fibers may be obtained from plant material such as from the seeds, stem and/or leaves of plants. Preferably, the feedstock comprises natural fibers obtained from plant material, such as linen, cotton, hemp, jute, flax, bamboo, sisal, coir and/or ramie.

In another preferred embodiment, the feedstock comprising textile material comprises synthetic cellulose fibers into value-added products. An example of a synthetic cellulose fiber is viscose and rayon. Viscose and rayon fabric are both manufactured from cellulose fibers that are extracted from wood pulp. Viscose and rayon may be produced in a number of ways, but are prepared from cellulose fibers, regardless of the manufacturing process.

Alternatively or additionally, the method according to the invention is particularly suitable for converting a feedstock comprising a paper material. Yearly, about 85 million tons of paper waste is created. Of this waste, only part is recycled or otherwise reused, the remaining being dumped in landfills, where it makes up about 16% of the solid waste. Hence, there is a large amount of paper-derived waste that is currently not properly decomposed, having adverse environmental effects. Accordingly by using paper material as a feedstock for use in the method according to the invention, adverse side-effects to the environment currently associated with decomposition of paper waste, may be avoided. Therefore, the feedstock for use in the method according to the invention preferably comprises paper, paperboard and/or cardboard.

The feedstock comprising textile material and/or paper material as defined in the method according to the invention comprises cellulose. Preferably, the content of cellulose in a feedstock comprising textile material and/or paper material is at least 40 wt.%, more preferably at least 50 wt.%, at least 60 wt.%, at least 70 wt.%, at least 80 wt.%, at least 90 wt.%, at least 95 wt.% of cellulose. For instance, the feedstock may comprise between 40 and 100 wt.%, preferably between 50 and 90 wt.%, more preferably between 60 and 85 wt.%, based on the total weight of the feedstock. Said feedstock further preferably comprises hemicellulose, preferably in a content of 5 to 25 wt.%, more preferably between 10 and 20 wt.%, in particular between 12 and 18 wt.%, based on the total weight of the feedstock. Said feedstock comprising textile material and/or paper material further preferably comprises lignin, preferably in a content of between 0.5 and 20 wt.%, more preferably between 2 and 15 wt.%, in particular between 3 and 10 wt.% of lignin.

Particularly preferred in a method according to the invention, is a feedstock comprising textile and/or paper material, wherein said feedstock comprises between 40 and 99 wt.% of cellulose, preferably between 65 and 90 wt.% of cellulose, more preferably between 70 and 85 wt.% of cellulose, between 0.5 to 25 wt.% hemicellulose, preferably between 5 and 20 wt.% of hemicellulose, more preferably between 10 and 15 wt.% of hemicellulose and between 0.5 and 20 wt.% of lignin, preferably between 2 and 10 wt.%, more preferably between 5 and 8 wt.% of lignin. Said feedstock comprising textile material and/or paper material may optionally comprise between 0-15 wt.% of one or more further components such as wax, pectin, ash and/or protein.

The inventor surprisingly realized that the method according to the invention is particularly suitable for converting a feedstock comprising textile material and/or paper material that has a relatively low cellulose content, preferably a cellulose content of less than 80 wt.%, more preferably less than 75 wt.%, more preferably less than 70 wt.%, in particular less than 65 wt.%, based on the total weight of the feedstock.

Feedstocks comprising textile and/or paper materials having a low cellulose content are notoriously difficult to convert to value-added products without using harsh chemical or physical treatment steps, due to their high recalcitrance towards known fermentation methods. Without wishing to be bound by theory, it is believed that in textile materials comprising less than 80 wt.% of cellulose, preferably less than 70 wt.%, the cellulose fibers are not very well accessible to the fermenting micro-organisms, because they are incorporated into larger composite materials, such as in lignocellulose.

The inventor surprisingly found that the induced fungal cell culture is capable to adapt in such a way that an enzyme mixture suitable to convert said textile material comprising less than 80 wt.% of cellulose into value-added products is produced in the method according to the invention, allowing conversion of said textile materials comprising less than 80 wt.% of cellulose into value-added products.

Examples of feedstocks comprising a cellulose content of less than 80 wt.% include but are not limited to hemp, jute, sisal, flax, linen, ramie, bamboo and coir.

Therefore, the feedstock for use in a method according to the invention preferably comprises a textile material selected from the group consisting of hemp, jute, sisal, flax, linen, ramie, bamboo, coir and combinations thereof.

In a method according to the invention, the feedstock comprising textile and/or paper material may comprise one or more non-textile and non-paper components, such as a dye, a water-proofing agent and/or a lubricant.

According to the invention, said feedstock comprising textile material and/or paper material preferably comprises or consists of comminuted textile material and/or paper material. Said textile and/or paper material may be comminuted using any method known in the art, such as cutting, grinding, milling, chopping, mincing or ripping of the textile material into smaller pieces. The textile material and/or paper material is preferably comminuted to a particle size of 0.5 to 5 mm, preferably 1 to 3 mm, most preferably 1.5 to 2.5 mm. Such particle size allows good accessibility of the induced fungal cell culture to the comminuted feedstock, hence improving the efficiency of the method according to the invention.

The feedstock comprising textile material and/or paper material is converted into one or more value-added products. Preferably, said value-added product is selected from the group consisting of fermentable sugars, bioethanol, cellulosic nanofibers, amino acids, peptides, biogas or a combination thereof.

These value-added products are characterized in that they have an economical, scientific, nutritional, or mechanical purpose. Preferably, said value-added products may be used in the food and drinks industry, the transport industry such as the automotive industry or aircraft industry, the materials industry, such as the textile industry, the paper industry and/or the food and drinks industry.

The inventor surprisingly realized that with the method according to the invention, feedstocks comprising textile material and/or paper material may be converted into ethanol in one step, i.e. without requiring the use of yeast or acidogenic bacteria converting fermentable sugars to ethanol. This is advantageous, because it allows the production of biofuels from a feedstock comprising textile material and/or paper material in one step, hence significantly improving efficiency and reducing costs.

It was further found that the method according to the invention allows the formation of cellulosic nanofibers. Cellulosic nanofibers are fibers of cellulose that have an average length of between 10 and 900 nm, preferably between 50 and 750 nm, more preferably between 100 and 600 nm, in particular between around 250 and 500 nm. Said nanofibers were found to be particularly suitable to for use in high quality paper, due to a superb resistance to ageing, higher strength and improved flexibility compared to regular paper.

The fungal cell as used in the method according to the invention may be a fungal cell of any of the genera *Myceliophthora, Sporotrichum, Thielavia, Corynascus, Chrysosporium* and/or *Ctenomyces*

Examples of species of the genus *Myceliophtora* are *Myceliophthora thermophila, Myceliophthora lutea, Myceliophthora vellerea, Myceliophthora hinnulea, Myceliophthora novoguineesis, Myceliophthora sepedonium, Myceliophthora fergusii, Myceliophthora similis* and *Myceliophthora heterothallica.*

Examples of species of the genus *Sporotrichum* are *Sporotrichum anglicum, Sporotrichum asteroids, Sporotrichum beurmannii, Sporotrichum carougeaue, Sporotrichum councilmani, Sporotrichum equi, Sporotrichum fonsecae, Sporotrichum grigsbyi, Sporotrichum indicum, Sporotrichum jeanselmei, Sporotrichum pannorum, Sporotrichum pruinosum, Sporotrichum pulverulentum, Sporotrichum schenckii, Sporotrichum thermophilum and Sporotrichum tropicale.*

Examples of species of the genus Thielavia are *Thielavia appendiculata, Thielavia arenaria, Thielavia australiensis, Thielavia basicola, Thielavia coactilis, Thielavia dacrydioides, Thielavia fragilis, Thielavia heterothallica, Thielavia hyalocarpa, Thielavia hyrcaniae, Thielavia inaequalis, Thielavia intermedia, Thielavia kuwaitensis, Thielavia microspora, Thielavia minuta, Thielavia ovispora, Thielavia pallidospora, Thielavia peruviana, Thielavia subthermophila, Thielavia terrestris, Thielavia terricola, Thielavia tortuosa* and *Thielavia wareingii.*

Examples of the species of the genus *Corynascus* are *Corynascus sepedonium, Corynascus similis, Corynascus verrucosus, Corynascus novoguineesis, Corynascus thermophilus, Corynascus citrinus, Corynascus fuminmontanus, Corynascus sexualis* and *Corynascus heterothallicus.*

Examples of species of the genus *Chrysosporium* include *Chrysosporium baduri, Chrysosporium botryoides, Chrysosporium carmichaelii, Chrysosporium europae, Chrysosporium filiforme, Chrysosporium georgiae, Chrysosporium globiferum, Chrysosporium hispanicum, Chrysosporium holmii Chrysosporium inops, Chrysosporium keratinophilum, Chrysosporium kreiselii, Chrysosporium littoralis Chrysosporium lobatum, Chrysosporium longisporum, Chrysosporium. lucknowense Chrysosporium. medium, Chrysosporium. mephiticum, Chrysosporium merdarium, Chrysosporium minor, Chrysosporium. pannicola, Chrysosporium pilosum, Chrysosporium pseudomerdarium, Chrysosporium pyriformis, Chrysosporium queenslandicum, Chrysosporium roseum, Chrysosporium siglerae, Chrysosporium sulfureum, Chrysosporium synchronum, Chrysosporium tropicum, Chrysosporium. undulatum, Chrysosporium vallenarense, Chrysosporium vespertilii, Chrysosporium xerophilum* and *Chrysosporium zonatum*

Examples of species of the genus *Ctenomyces* include *Ctenomyces serserratus, Ctenomyces peltricolor, Ctenomyces albus, Ctenomyces obovatus and Ctenomyces vellereus.*

Preferably, the fungal cell as used in the method according to the invention is a fungal cell of the genus *Myceliophthora,* more preferably a fungal cell of *Myceliophthora thermophila,* even more preferably a fungal cell of *Myceliophthora thermophila* DSM 1799. *M. thermophila* is also referred to as *Chrysosporium thermophilum, Sporotrichum thermophile, Thielavia heterothallica* or *Corynascus heterothallicus.*

Fungal cells of the genus *Myceliophtora,* preferably *M. thermophila* advantageously were found to efficiently convert feedstocks comprising textile material and/or paper material into one or more value-added product(s). Further, fungal cells of the genus *Myceliophtora,* preferably *M. thermophila* are easy to handle, and may be cultured under a broad range of conditions, such as different temperature and pH conditions.

Optionally, said fungal cell may comprise a genetic modification, for example a natural modification or a modification directed to increasing the ability of said fungal cell to produce one or more enzymes involved in converting textile materials into value-added products. Preferably, said fungal cell is not genetically engineered.

In the method according to the invention, the fungal cells are cultured in a liquid growth medium comprising at least 0.3 wt.% of lactose, preferably between about 0.4 to about 0.8 wt.%, at least 0.1 wt.% of a nitrogen source, preferably between about 0.2 to about 0.8 wt.%, and less than 2 wt.% of a non-inducing carbon source, preferably less than 1 wt.%, more preferably less than 0.5 wt.%, less than 0.4 wt.%, less than 0.3 wt.%, less than 0.2 wt.%, less than 0.1 wt.%, most preferably essentially free of a non-inducing carbon source.

Said liquid growth medium ensures sufficient growth of the fungal cells, due to the presence of lactose as a carbon source and a nitrogen source that enables the fungal cells to grow, whilst simultaneously inducing the expression of one or more cellulase and optionally one or more other enzymes involved in degradation of the feedstock comprising textile material and/or paper material.

Although lactose is typically not a preferred carbon source for fungal growth, in particular growth of cells of the genera *Myceliophthora, Sporotrichum, Thielavia, Corynascus, Chrysosporium* and/or *Ctenomyces,* the inventor realized that said fungal cells are sufficiently capable of growing on lactose as carbon source to provide an induced fungal cell culture suitable for efficiently converting textile material and/or paper material into value-added products. In this context, growth of fungal cells may be determined using any suitable method known in the art. For example it may be determined by visual inspection, or it may be quantified by determining the intracellular protein content using the Bradford method (Bonjoch et al. 2001. Hanbook of Plant Ecophysiology Techniques, Chapter 19, Kluwer Academic Publishers, 283-295). Preferably, in step (i) of the method according to the invention, the induced fungal cell culture has a dry weight of at least 1 mg/mL liquid growth medium, more preferably at least 1.5, at least 1.6, at least 1.7, at least 1.8, at least 1.9, in particular about 2 mg/mL. With such contents of induced fungal cell culture in the medium, it was found that the conversion of the feedstock comprising textile material and/or paper material into one or more value-added product(s) was most efficient.

Typically the induced fungal cell culture as defined in the method according to the invention, has a cellulase activity of at least 1 µmoles, preferably at least 2 µmoles, at least 2.5 µmoles, at least 3 µmoles, at least 3.5 µmoles, at least 3.6 µmoles, at least 3.7 µmoles, at least 3.8 µmoles of reducing sugar produced per milligram protein per minute. Said cellulase activity may be determined using any suitable method known in the art, for example using the method described by Wood et al., 1988. Methods in Enzymology 160, Biomass Part A: Cellulose and Hemicellulose, eds, Academic Press Inc. and Ghose, 1987. Pure & Appl. Chem. 59(2):257-268.

The liquid growth medium as defined in the method according to the invention further comprises a nitrogen source. Examples of nitrogen sources suitable for use in the method according to the invention include a source of ammonia, such as ammonium acetate, ammonium sulfate, ammonium chloride and ammonium nitrate, a source of nitrate, such as potassium nitrate, ammonium nitrate, and sodium nitrate, a source of nitrite, such as potassium nitrite and sodium nitrite, a source of amino acids, such as glutamine, and a source of organic nitrogen such as urea, uric acid, amines, amides, purines, and/or pyrimidines. The presence of a nitrogen source is required for fungal growth.

The liquid growth medium as defined herein, for use in the method according to the invention, comprises less than 2 wt.% of a non-inducing carbon source. Examples of non-inducing carbon sources are sucrose, fructose, galactose and glucose. Therefore, in a method according to the invention, the liquid growth medium is preferably essentially free of sucrose, fructose, galactose and/or glucose.

The absence of substantial amounts of one or more non-inducing carbon sources stimulate the fungal cells, in order to survive, to metabolize lactose and optionally one or more additional inducing carbon sources, because there is no other carbon-source available. Such a growth medium therefore stimulates the production of extracellular cellulases that play an important role in the conversion of textile materials and/or paper material into value-added products.

The liquid growth medium as defined herein, may therefore optionally comprise one or more additional inducing carbon-source apart from lactose. Such an inducing carbon source is preferably an oligosaccharide, i.e. carbohydrates comprising 2 to 20 monomer units, wherein the monomer units are linked via β-1,4-linkages to one another. Examples of inducing carbon-sources include carboxymethyl cellulose, filter paper, lignocellulosic material and cellobiose.

The liquid growth medium as defined in the method according to the invention may comprise one or more conventional trace elements, such as zinc, manganese, molybdenum and copper. These trace elements may promote growth of fungal cells.

Said liquid growth medium preferably comprises buffer system to ensure an essentially stable pH during cultivation of fungal cells. Preferably, the liquid growth medium as defined in the method according to the invention comprises a phosphate buffer system.

Optionally, in accordance with the invention, the pH of the liquid growth medium may be adjusted, e.g. using a suitable acidic or alkaline stock solution, prior to or during the culturing of the fungal cells. Preferably, the pH of the liquid medium is between 5.5 to 8, more preferably between 6.0 to 7.5, in particular between 6.5 and 7.0. These conditions have been found to be particularly suitable for inducing fungal cells in the method according to the invention.

The liquid growth medium preferably comprises lactose, urea, sodium sulfate, ammonium acetate, a phosphate buffer system and conventional trace elements, more specifically, based on the weight of the liquid growth medium,
- about 0.2 to about 0.8 wt.% of lactose;
- about 0.1 to about 0.7 wt.% of ammonium acetate;
- about 0.1 to about 0.7 wt.% of urea;
- less than 1 wt.% of non-inducing carbon source.

According to the method of the invention, the liquid growth medium may optionally be subjected to one or more processing steps, such as dilution with water, filtration, centrifugation, sterilization or a combination of these processes, prior to cultivation of the fungal cells.

Said fungal cells are preferably cultured for a period of at least 24 hours, more preferably at least 36 h, at least 48 h, at least 72 h or at least 96 h. Such a culturing time was found to be sufficient to obtain an induced fungal cell culture.

Said fungal cells are preferably cultured at a temperature of between 20 °C and 70 °C, preferably between 30 °C and 60 °C, more preferably between 40 °C and 55 °C, in particular between 45 °C and 52 °C.

Further, said fungal cells may be cultured under aerobic or anaerobic conditions. For example, the process may be performed aerobically such that air or oxygen gas is provided to the liquid growth medium at a rate of from about 0.5 to about 5 gas volume flow per unit of liquid volume per minute (vvm), preferably about 1 to about 4 vvm, in particular about 2 to 3 vvm. Optionally, antifoaming agents (either silicone, or non-silicone based) may be added to control excessive foaming during the process as required and as is known to one of skill in the art.

Further, said fungal cells may optionally be cultured under agitation at a sufficient rate to ensure distribution of the cells throughout the medium and to prevent formation of concentration gradients of nutrients. For example, the culture may be agitated by shaking from about 100 to about 1000 rpm, preferably about 150 to 500 rpm, more preferably about 200 to 300 rpm.

In the method according to the invention, the feedstock comprising textile materials and/or paper material is optionally subjected to a sterilization treatment (step ii), to remove undesired micro-organisms such as bacteria from the feedstock comprising textile material and/or paper material. It is beneficial to remove micro-organisms from the feedstock to control the conversion of the textile material and/or paper material to value-added products. In absence of sterilization, undesired micro-organisms such as bacteria may be present which may compete with the induced fungal cell culture, e.g. causing impaired growth or killing part of the induced fungal cell culture, or forming undesired side-products which contaminate the fermentation mixture.

Sterilization may be achieved using any method known in that art that is suitable of removing micro-organisms. In principle, any known method of sterilization is suitable herein, provided said sterilization removes micro-organisms such as bacteria that are present in the feedstock comprising textile material and/or paper material. Examples of methods of sterilization include steam sterilization, sterilization using dry heat, sterilization using chemicals and sterilization by radiation.

A preferred method of sterilization is treatment with steam, preferably at a temperature between 100 and 150 °C, more preferably between 105 and 140 °C, even more preferably between 110 and 130 °C, in particular between 115 and 120 °C for a duration of at least 5 minutes, preferably at least 10 minutes, at least 15 minutes, in particular at least 30 minutes.

Sterilization using steam according to the method of the invention may be achieved under pressure, preferably at least 15 psi, more preferably at least 20 psi, in particular at least 30 psi. Sterilization using steam may for example be carried out in an autoclave.

An advantage of using steam, preferably steam at a temperature between 100 and 150 °C, more preferably between 105 and 140 °C, even more preferably between 110 and 130 °C, in particular between 115 and 120 °C, is that the feedstock comprising textile material becomes soaked in water. Feedstocks which are soaked in water aid the conversion of textile materials and/or paper material to value-added products when contacted with the induced fungal cell culture, because the accessibility of the feedstock comprising textile material and/or paper material and the induced fungal cell culture is improved.

A further advantage of using steam is that it is easily accessible, does not require high costs, harsh chemicals or complex equipment.

Alternatively or additionally, according to the invention, the feedstock may be sterilized at a pH of between 4 and 8, preferably between 5 and 7.5, more preferably between 6.5 and 7.3.

In the method according to the invention, the sterilized feedstock is contacted with the induced fungal cell culture and fermentation medium to form a fermentation mixture (step iii). Said fermentation medium is preferably a liquid growth medium, preferably a liquid growth medium comprising at least 0.3 wt% of lactose, at least 0.1 wt% of a nitrogen source, and less than 2 wt% of a non-inducing carbon source.

Preferably, said fermentation medium is the same medium as the liquid growth medium as defined in step (i) of the method according to the invention.

Alternatively, said fermentation medium may be essentially free of a carbon-source, to promote conversion of the textile material and/or paper material into value-added product(s). Such a fermentation medium may comprise a nitrogen source, such as a source of ammonia, such as ammonium acetate, ammonium sulfate, ammonium chloride and ammonium nitrate, a source of nitrate, such as potassium nitrate, ammonium nitrate, and sodium nitrate, a source of nitrite, such as potassium nitrite and sodium nitrite, a source of amino acids, such as glutamine, and a source of organic nitrogen such as urea, uric acid, amines, amides, purines, and/or pyrimidines, and a buffer, such as a phosphate buffer, and optionally one or more trace elements.

Accordingly, the fermentation medium as defined in step (iii) of the method according to the invention may preferably comprise about 0.1 wt.% to 2 wt.% of a nitrogen source, less than 2 wt.%, preferably less than 1 wt.% of a carbon source and a buffer.

Said contacting may be achieved using any suitable method known in the art, for example by transferring the fermentation medium and the induced fungal cell culture into a suitable container, such as a sterilized Erlenmeyer flask, using a suitable transportation means, such as a pipette. Said sterilized feedstock may then be added using a suitable transportation means, e.g. tongs, pincers or the like.

In the method according to the invention, the order of adding said sterilized feedstock, said fermentation medium and said induced fungal cell culture is not essential. Hence, according the method according to the invention, the sterilized feedstock may be charged first to a suitable container, followed by the fermentation mixture and then the induced fungal cell culture, or followed by the induced fungal cell culture and then the fermentation medium. Alternatively, the fermentation medium may be charged first to a suitable container, followed by the induced fungal cell culture and then the sterilized feedstock, or followed by the sterilized feedstock and then the induced fungal cell culture. Alternatively, the induced fungal cell culture may be charged first to a suitable container, followed by fermentation medium and then the sterilized feedstock, or followed by the sterilized feedstock and then the fermentation medium.

Preferably, according to the invention, the sterilized feedstock may be immerged in a liquid medium, preferably a liquid growth medium comprising at least 0.3 wt.% of lactose, at least 0.1 wt% of a nitrogen source and less than 1 wt.% of a non-inducing carbon source, and left to soak for at least 0.5 h, preferably at least 1 h, prior to contacting the soaked sterilized feedstock with the fermentation medium and the induced fungal cell culture. Advantageously, a soaked sterilized feedstock was found to be well accessible for the induced fungal cell culture, thereby increasing the conversion rate of the textile material into the value-added product.

In the method according to the invention, the fermentation mixture is preferably incubated under conditions allowing for conversion of the textile material into value-added product(s) (step iv).

Preferably, in the method according to the invention, the fermentation mixture is incubated at a temperature of between 20 °C and 70 °C, preferably between 30 °C and 60 °C, more preferably between 40 °C and 55 °C, most preferably between 45 °C and 52 °C, in particular around 50 °C. It was found that the sterilized feedstock comprising textile material is efficiently converted at this temperature range.

Preferably, in the method according to the invention, the fermentation mixture is incubated under rotation at about 50 to about 400 rounds per minute (rpm), more preferably about 100 to about 300 rpm, even more preferably about 125 to about 250 rpm, in particular about 150 to about 200 rpm. Such rotation promotes a homogenous distribution of the induced fungal cell culture and sterilized feedstock in the fermentation mixture, thereby improving the efficiency of the method.

According to the invention, the fermentation mixture is preferably incubated at a pH of between 4 and 8, more preferably between 5 and 7.5, in particular between 6.5 and 7.3.

During incubation the induced fungal cell culture is capable of adapting to efficiently convert the feedstock comprising textile material and/or paper material. Hence, in the method according to the invention, the induced fungal cell culture expresses those enzymes that are required to convert the textile material and/or paper material into one or more value-added product(s).

Without wishing to be bound by any theory, it is believed that the induced fungal cell culture is capable of excreting one or more cellulases. The term "cellulase" or "cellulase enzymes" as used herein, refers to enzymes that catalyse the hydrolysis of the 1,4-glucosidic bonds connecting glucose monomer units in the cellulose polymer. Examples of cellulases include *endo*-1,4-β-D-glucanases (E.C. 3.2.1.4), *exo*-1,4-β-D-glucanases (E.C.3.2.1.74), cellobiohydrolases (E.C. 3.2.1.91), and β-glucosidase (E.C. 3.2.1.21). Endoglucanases hydrolyze accessible glucosidic bonds in the middle of the cellulose chain, while cellobiohydrolases release cellobiose from these chain ends processively. β-glucosidases hydrolyze cellobiose to glucose and, in doing so, minimize product inhibition of the cellobiohydrolases.

The induced fungal cell culture for use in the method according to the invention may further express one or more hemicellulase enzymes. A hemicellulase, or hemicellulose degrading enzyme, is an enzyme capable of hydrolysing the glycosidic bonds in a hemicellulose polymer. Hemicellulases include, but are not limited to, xylanase (E. C. 3.2.1.8), beta-mannanase (E.C. 3.2.1.78), alpha-arabinofuranosidase (E.C. 3.2.1.55), beta-xylosidases (E.C. 3.2.1.37), and beta-mannosidase (E.C. 3.2.1.25). Hemicellulases typically comprise a catalytic domain of Glycoside Hydrolase Family 5, 8, 10, 11, 26, 43, 51, 54, 62 or 113.

In the method according to the invention, the induced fungal cell culture may further express one or more lignin-degrading enzymes. Lignin-degrading enzymes are enzymes that oxidize and participate in the depolymerisation of lignin and include, for example, laccases (E.C. 1.10.3.2), lignin peroxidases (E.C. 1.11.1.14), manganese peroxidases (E.C. 1.11.1.13) and cellobiose dehydrogenases (E.C. 1.1.99.18).

In addition, the induced fungal cell culture may express one or more additional enzymes such as pectinases, pectate lyases, galactanases, amylases, glucoamylases, glucuronidases, and galacturonidases.

In the method according to the invention, the fermentation mixture is preferably incubated for at least 1 h, preferably at least 24 h, more preferably at least 48 h, at least 72 h, at least 96 h, at least 120 h, at least 144 h, in particular at least 168 h. The incubation time in the method according to the invention may be adapted according to the amount of feedstock that is present in the fermentation mixture and depending on the rate of the feedstock comprising textile material and/or paper material is converted into one or more value-added product(s).

In the method according to the invention, the value-added products are optionally isolated from said fermentation mixture. Isolation may be achieved using any method known in the art, such as by separating the solid phase from the fluid phase by centrifugation followed by decanting or pipetting of the fluid phase, or by filtration of the fermentation mixture over a suitable filter. The skilled person is aware of methods for isolating value-added products.

The method according to the invention may be performed batch wise, continuously or semi-continuously. Preferably, the method is carried out continuously, e.g. by allowing addition of the feedstock comprising textile and/or paper material simultaneously with extraction of fermentation mixture comprising the one or more value-added product(s). Such a method allows control of the formation of the one or more value-added product(s) to a certain extend and improves efficiency of the method, since no lag time between the batches is present.

The value-added product may optionally be subjected to one or more purification steps to separate undesired products. For example, the value added product may be purified using (vacuum) distillation, column chromatography, HPLC or crystallization.

With the method according to the invention, said value-added product is preferably obtained in a high yield, preferably at least 80%, more preferably at least 85%, at least 90%, at least 95%, at least 96%, at least 97%, at least 98%, at least 99%, in particular substantially free of impurities, i.e. wherein impurities are not detectable with common analytical techniques known at the date of filing.

For the purpose of clarity and a concise description, features are described herein as part of the same or separate embodiments, however, it will be appreciated that the scope of the invention may include embodiments having combinations of all or some of the features described.

The invention is illustrated by the following non-limiting example.

### Example 1: Production of value-added products from cotton, paper, hemp, viscose or denim.

### 1.1 Materials

HPLC-grade sulphuric acid was purchased from Biosolve, The Netherlands.

Lactose, Na₂SO₄, CH₃COONH₄, K₂HPO₄, KH₂PO₄, FeSO₄ ·7H₂O, MgSO₄ ·7H₂O, EDTA, C₆H₆Na₂O₇, ZnSO4 ·7H₂O, CaCl₂ ·2H₂O, MgSO₄ H₂O, H₃BO₃, CuSO₄ ·5H₂O, Na₂MoO ·2H₂O, and CoSO₄ ·7H₂O were purchased from Sigma Aldrich.

*Myceliophthora thermophila* DSM 1799 and *Rasamsonia emersonii* DSM 2432 and *Chaetomium thermophilum* used in this study were purchased from Leibniz Institute DSMZ-German Collection of Microorganisms and Cell Cultures GmbH, Germany. Thermo Scientific *Penicillium chrysogenum* NRRL and *Penicillium chrysogenum* WISCONSIN were kindly provided by University of Groningen, The Netherlands.

### 1.2 Conversion of feedstock comprising cotton, paper, hemp, viscose or denim into value-added products

One liter of liquid growth medium was prepared by mixing the following reagents (in g/l); lactose, 13; urea 4,0; Na₂SO₄, 4.0;CH₃COONH₄,5.0; K₂HPO₄, 2,12; KH₂PO₄, 5.1; supplemented with 10 ml of a trace element solution (in g/l): FeSO₄·7H₂O, 24.84; MgSO₄·7H₂O, 0.0125; EDTA, 31.25; C₆H₆Na₂O₇,43.75; ZnSO4 ·7H₂O, 2.5; CaCl₂ ·2H₂O, 1.6; MgSO₄ ·H₂O, 3.04; H₃BO₃, 0.0125; CuSO₄ ·5H₂O, 0.625; Na₂MoO ·2H₂O, 0.0125; CoSO₄ ·7H₂O, 0.625. The medium was adjusted to pH 6.5.

Fresh spores of the strains *M. thermophila* DSM 1799, *R. emersonii* DSM 2432, *C. termophilum* and *P. chrysogenum* NRRL and *P. crysogenum* WISCONSIN were inoculated in the liquid growth medium and cultured in a shaking incubator at 150 rpm for 48 h at 45°C to obtain an induced fungal cell culture.

A feedstock comprising, respectively, cotton, paper, viscose or denim material was subjected to particle size reduction by grinding and/or knife milling to particles < 2 mm. This is shown in Figure 1B (cotton); Figure 3B (denim); Figure 8B (paper) and Figure 10B (viscose). Hemp bast and shive fibers were not subjected to knife milling or grinding and were directly used for further experimentation.

The (grinded) feedstocks were autoclaved at 121°C for 15 min. and then incubated with the induced fungal culture and liquid growth medium in a rotary incubator at 150 rpm at 45°C for 46 days. A control experiment was performed, wherein the feedstocks were incubated in the liquid growth medium for 46 days at the same conditions.

The production of value-added products and growth of the fungal cell culture and were monitored over time (see sections 1.2.1 and 1.2.2.)

### 1.2.1 Determination of metabolite production

The supernatants of fermentation mixtures comprising induced cells of the strains *Penicillium chrysogenum* NRRL, WISCONSIN, *Myceliophthora thermophila* or *Rasamsonia emersonii* and a feedstock comprising textile material or paper, were analyzed after 3, 7, 14, 20, 27, 46 days of growth by means of high performance liquid chromatography (Shimadzu, Kyoto, Japan) using an Aminex HPX-87H column at 65°C (Bio-RAD). The mobile phase was 0.005 N H₂SO₄ at a flow rate of 0.55 ml/min. A refractive index detector (Shimadzu, Kyoto, Japan) was used to measure the concentrations of D-glucose, D-xylose, acetic acid, glycerol and ethanol. The levels of glucose, xylose, acetic acid, glycerol and ethanol were determined with an Accella1250 HPLC system using an Aminex HPX-87H column at 60°C (Bio-RAD) coupled with the ES-MS Orbitrap Exactive (Thermo Fisher Scientific, CA, USA). Triplicate shake flasks cultivations were performed for each strain. Samples were centrifuged (14,000 x g for 5 minutes) and 50 µL of supernatant was transferred to an autosampler vial. The results are shown in Figure 1C, Figure 3C, Figure 5B, Figure 8C, Figure 10C and Figures 13-18.

As can be derived from Figures 13-18, the levels of glucose and xylose in the supernatant of the fermentation mixture comprising induced cells of *M. thermophila* DSM 1799 and a feedstock were approximately 1.5 to 2-fold higher compared to the control experiment (lacking the induced *M. thermophila* cell culture), in each experiment. The presence of glucose in the control medium can be explained by spontaneous hydrolysis of lactose into glucose and galactose. For feedstocks comprising cotton and viscose, the levels of glucose were approximately 1.5 fold higher after 3, 7, 14, 20, 27 and 46 days of the experiment. The presence of xylose could be measured up to 20 days of the experiment and was absent in the control experiment (Figures 13 (cotton) and Figure 18 (viscose)). This indicates that hemicellulose was no longer present in the fermentation mixture after 20 days.

For the feedstock comprising denim, the level of glucose was approximately 1.5-2.5-fold higher after 3, 7 and 14 days, and about 1.2 fold higher after 20 and 27 days. After 46 days, no significant difference in glucose levels were obtained compared to the control. The presence of xylose could be detected up to 14 days of the experiment and was absent in the control experiment (Figure 14). This indicates that hemicellulose was no longer present in the fermentation mixture after 14 days.

For the feedstock comprising paper the level of glucose was approximately 1.5-2.5-fold higher after 3, 7, 14, 20 and 27 days. After 46 days, no significant different in glucose levels were obtained compared to the control. The presence of xylose could be detected up to 27 days of the experiment, indicating that hemicellulose was completely degraded after 27 days. Xylose was absent in the control experiment (Figure 17).

For the feedstock comprising hemp the level of glucose was approximately 1.5-2.5-fold higher after 3, 7, 14, 20, 27 and 46 days. The presence of xylose could be detected up to 27 days of the experiment, indicating that there was no hemicellulose present in the fermentation mixture after 27 days. It was absent in the control experiment (Figure 15).

Further, as shown in Figure 16, when the hemp bast and shives fibers were cultured separately with induced *M. thermophila* cells in a liquid growth mixture, traces of glycerol could be detected up to 18 days for a feedstock comprising hemp bast fibers, and xylose could be detected up to 5 days for a feedstock comprising hemp shive fibers.

The results demonstrate that strain *M. thermophila* DSM 1799 is able to convert the feedstock comprising cotton, paper, hemp, viscose or denim into glucose and xylose (Figures 13-18). Fluctuations in the lactose/glucose profiles in the control experiment may be explained by evaporation of the liquid growth medium.

To the contrary, strains *R. emersonii, P. chrysogenum* NRRL and *P. chrysogenum* WISCONSIN, did not significantly convert the feedstock comprising cotton. (Figures 12A and 12B). Although these strains were known to be capable of expressing cellulases, they were evidently not capable of converting cotton into value-added product(s). This supports the surprising finding that fungal cells of the genus *Myceliophthora, Sporotrichum, Thielavia, Corynascus, Chrysosporium, Ctenomyces,* in particular of *Mycelophtohora,* specifically *M. thermophilus* is capable of growing on textile and/or paper material, as well as adapting to excrete a cocktail of enzymes required for conversion of said textile and/or paper material.

The strain *Chaetomium thermophilum* did not grow on the liquid growth medium and was therefore excluded from the experiment (Figure 12C).

### 1.2.2. Determination of cell growth

On day 0, the induced fungal cell culture was inoculated in the liquid growth medium containing the sterilized feedstock and grown for 17 days in a rotary incubator at 150 rpm at 45°C. Duplicates of shake flask cultivations were performed. A control experiment was performed using *M. thermophila* DSM 1799 in a liquid growth medium in the absence of the sterilized feedstock.

Cells were harvested and sonicated in lysis buffer (50mM Tris/HCl pH 7.6; 0.5M NaCl; 10 mM imidazole). The intracellular protein concentration was determined using Bradford method as previously described (Bonjoch et al. 2001. Hanbook of Plant Ecophysiology Techniques, Chapter 19, Kluwer Academic Publishers, 283-295). The supernatant of a fermentation mixture comprising induced *M. thermophila* cells and a sterilized feedstock comprising cotton or paper respectively in a liquid growth medium, was analyzed after 1, 3, 5, 7, 9, 11, 13, 15 and 17 days by means of Bradford method.

As shown in Figure 20, the protein concentration in the culture medium of *M. thermophila* in presence of cotton remained stable for the first seven days and increased by approximately 1.5- to 2- fold after 8-9 days. It remained stable until 17 days in the experiment. The protein concentration in the control experiment, lacking the feedstock, decreased over time due to a decrease in nutrients.

Similarly, the protein concentration of the culture medium of *M. thermophila* in the presence of paper steadily increased by approximately 1.5- to 2- fold over time (Figure 22). The protein concentration in the control experiment decreased over time due to a decrease in nutrients.

The levels of lactose and glucose were monitored over time by HPLC, showing that the lactose content provided by the liquid growth medium decreased whereas glucose levels increased, as a result of conversion of the feedstock (Figure 19 (cotton) and Figure 21 (paper)).

In conclusion, the cell growth profile indicates that *Myceliophthora thermophila* DSM 1799 utilizes the feedstocks comprising cotton or paper as nutrient source as reflected in an increased intracellular protein concentration in the fermentation mixture overtime (Figures 20 and 22). This is confirmed by detection of xylose (breakdown product of hemicellulose) and glucose (breakdown product of cellulose) over time (Figure 19 and 21).

## Claims

1. A method for converting a feedstock comprising textile material and/or paper material into one or more value-added product(s), comprising the steps of:
(i) culturing fungal cells of the genus *Myceliophthora, Sporotrichum, Thielavia, Corynascus, Chrysosporium, Ctenomyces* or any mixture thereof, in a liquid growth medium comprising at least 0.3 wt% of lactose, at least 0.1 wt% of a nitrogen source, and less than 2 wt% of a non-inducing carbon source for a period to provide an induced fungal cell culture;
(ii) optionally subjecting a feedstock comprising textile material and/or paper material to a sterilization treatment to provide a sterilized feedstock;
(iii) contacting said feedstock or said sterilized feedstock with the induced fungal cell culture and a fermentation medium to form a fermentation mixture;
(iv) incubating the fermentation mixture under conditions allowing for conversion of the textile material into value-added product(s); and optionally
(v) isolating the value-added product(s) from said fermentation mixture.

2. The method according to claim 1, wherein the liquid growth medium comprises about 0.4 to about 0.6 wt% of lactose, about 0.2 wt.% to about 0.8 wt.% of a nitrogen source, and less than 1 wt% non-inducing carbon source.

3. The method according to claim 1 or 2, wherein the liquid growth medium comprises, based on the weight of the liquid growth medium,
- about 0.3 to about 0.8 wt.% of lactose;
- about 0.1 to about 0.7 wt.% of ammonium acetate;
- about 0.1 to about 0.7 wt.% of urea;
- less than 1 wt.% of non-inducing carbon source.

4. The method according to any one of the preceding claims, wherein the sterilized feedstock is immerged in a liquid medium, preferably a liquid growth medium comprising at least 0.3 wt.% of lactose, at least 0.1 wt% of a nitrogen source and less than 1 wt.% of a non-inducing carbon source, and left to soak for at least 0.5 h, preferably at least 1 h.

5. The method according to any one of the preceding claims wherein the fungal cell is of the genus *Myceliophthora,* preferably *Myceliophthora thermophila,* more preferably *Myceliophthora thermophila* DSM 1799.

6. The method according to any one of the preceding claims, wherein the feedstock comprises or consists of comminuted textile material.

7. The method according to claim 6, wherein the textile material is comminuted to a particle size of 0.5-5 mm, preferably 1 to 3 mm.

8. The method according to any one of the preceding claims, wherein the feedstock comprises cotton, paper, paperboard, cardboard, denim, hemp, viscose, or a combination thereof.

9. The method according to any one of the preceding claims, wherein the feedstock comprises natural cellulosic fibers, such as linen, cotton, hemp, jute, flax, bamboo, coir, sisal and/or ramie.

10. The method according to any one of the preceding claims, wherein the feedstock comprises man-made cellulose fibers, such as viscose and/or rayon.

11. The method according to any one of the preceding claims, comprising sterilizing the feedstock using a steam treatment, preferably comprising exposure for a duration of at least 15 minutes to a temperature between 100 and 150 °C, more preferably between 105 and 120 °C.

12. The method according to any one of the preceding claims, wherein said value-added product(s) comprise fermentable sugars, nanofibers of cellulose and/or biofuel, such as ethanol.

13. The method according to any one of the preceding claims, wherein step (v) comprises incubating the fermentation mixture for at least 1 hour, preferably at least 1 day, more preferably at least 5 days, in particular at least 7 days.

14. The method according to any one of the preceding claims, wherein no organic solvents and/or conditions comprising a pH of at least 9 or less than 3, are used.

15. The method according to any one of the preceding claims, wherein the fungal cells are not genetically engineered.
